# EUROPEAN PATENT APPLICATION

(11) **EP 2 289 497 A1**
(43) Date of publication of application: **02.03.2011**
(21) Application number: 10185613.6
(22) Date of filing: 09.11.2006
(51) Int. Cl.: A61K 9/50, A61K 31/135

(54) **Once-daily administration of central nervous system drugs**

(30) Priority: 10.11.2005 US 735178 P
(62) Divisional of application: 06850465.3
(71) Applicant: Circ Pharma Research and Development Limited, Dublin 2 (IE)
(72) Inventor: Mulligan, Seamus, Roscommon (IE)
(74) Representative: Swinkels, Bart Willem

(57) **Abstract**

Delayed onset chronotherapeutic formulations of central nervous system (CNS) drugs are disclosed. The formulations comprise at least one CNS drug or pharmaceutically acceptable salt thereof that exhibits an *in vivo* elimination half-life of less than about 8 hours, wherein the formulation exhibits at least one *in vivo* parameter, at steady state following administration to a subject, chosen from: an initial lag in absorption from about 2 hours to about 6 hours; a peak-to-trough ratio greater than or equal to about 4:1; a percent fluctuation of greater than or equal to about 100%; and a minimum time cover of greater than or equal to 50% of Cₘₐₓ of at least 8 hours.

## Description

This application claims priority to U.S. Provisional Patent Application No. 60/735,178, filed November 10, 2005.

Chronotherapy involves the synchronization of drug exposure with the circadian pattern of disease symptoms or underlying physiological functions. Such therapies provide a more rational or targeted approach for treating a disease. In addition, chronotherapeutic formulations may improve patient compliance by permitting a once-daily administration that maintains a steady state and release of the drug resulting in continued delivery of therapeutic concentrations. Such once-daily formulations are desirable because patient compliance can be as high as 80%, while with twice-a-day and three times-a-day dosing, compliance levels fall to 60% and 40%, respectively. *See, e.g.,* Shilo, et al., Ann. Pharmacotherapy, 35(11):1339-42, 2001. Thus, chronotherapeutic dosage forms that reduce the frequency of administration can significantly improve the therapeutic outcome.

Some chronotherapeutic formulations have been designed to create a delay, or lag, in initial drug release that reportedly synchronizes the onset of drug absorption and exposure with risk periods. Such formulations have typically been described as having a lag time of from about 2 to about 8 hours following administration of a single dose.

Focusing on lag times, however, overlooks many other important parameters that impact the efficacy of a chronotherapeutic formulation. For example, a drug having a long elimination half-life may be formulated with a standard lag phase and also provide adequate coverage throughout the day, but may accumulate with repeated doses. In contrast, a drug having a short elimination half-life (*e.g*., less than 8 hours) will not achieve sustained therapeutic blood levels if it is formulated simply with a standard lag phase because it is cleared much more quickly from the subject's system. Thus, in the case of short elimination half-life drugs, additional parameters must be addressed to prepare suitable chronotherapeutic formulations. Such parameters include the drug absorption rate, the timing of peak concentrations, the duration of therapeutic blood levels, the elimination half-life of the drug, and the duration of the washout of blood levels necessary to achieve an optimal chronotherapeutic plasma profile suitable for repeated dosing. Evaluation of such parameters may be desirable, for example, in central nervous system ("CNS") chronic conditions such as chronic pain.

For example, for short half life drugs current once daily dosing requires reformulation as controlled release dosage forms where the absorption rate is extended to achieve a longer duration of systemic exposure at clinically effective plasma concentrations (i.e., 50% of Cₘₐₓ). Thus, a goal of current once-daily dosage forms of short half-life drugs may be a reduced peak-to-trough plasma concentration ratio (*e.g*., < 4 such as < 2), a reduced plasma concentration % fluctuation(Cₘₐₓ-Cₘᵢₙ/Cₘₐₓ x 100) (*e.g*., < 100% such as < 50%) and a greatly extended period of time cover at 50% maximal plasma concentrations (minimally 12 hours).

This approach to the formulation of a CNS drug with short-half life has resulted in a large number of controlled release formulations. For example, in the case of tramadol (a centrally acting analgesic), which has a half life of 6.3 hours and its active metabolite (O-demethylated so called M1), which has a half-life of 7.4 hours, controlled release formulations have been developed to achieve extended plasma concentrations and reduced peak-to-trough fluctuations.

Certain CNS active drug classes including centrally acting analgesics and opioids, however, are associated with the development of dependence, tolerance, and exhibit varied withdrawal symptoms. As such, the dependence, tolerance, and withdrawal implications of controlled release formulations for such compounds has not been considered in their design and profile.

For example, as with many CNS drugs, long-term continuous administration often results in tolerance or desensitization to the drug. As a result, ever increasing amounts of the drug must be administered to maintain therapeutic efficacy. Unfortunately, the amount of drug that may be administered is often dose-limited by adverse side-effects caused by the drug. Thus, the development of desensitization in a subject can ultimately eliminate important long-term therapeutic options for treating a particular CNS condition with these drugs.

In addition to problems with tolerance, constant exposure to many CNS drugs presents complications when the therapy is suddenly discontinued. This may occur, for example, when a subject does not have access to his or her medication, or when the drug administration must be halted for medical reasons (*e.g*., due to side-effects, negative interactions with other medications, surgical complications, *etc.*).

When CNS therapy is discontinued following a course of continuous treatment, subjects experience an "exaggerated rebound phenomenon" or varied withdrawal effects. Some signs and symptoms of withdrawal include rhinorrhea, lacrimation, yawning, chills, hyperventilation, hyperthermia, mydriasis, muscular aches, vomiting, diarrhea, anxiety, and hostility. Basic & Clinical Pharmacology 508 (Bertram G. Katzung, ed., 9th ed., 2004). The package insert for one commercially available analgesic (*e.g*., tramadol) also warns that panic attacks, severe anxiety, and paresthesias have been seen with abrupt discontinued use. See Package Insert, Ultram® (tramadol hydrochloride tablets) (Rev. May 2004).

Consequently, CNS drugs such as tramadol must be gradually reduced following a course of chronic administration. This caution, however, does not account for situations where cessation of treatment cannot be avoided (*e.g*., when a patient unexpectedly does not have access to the medication). Thus, the danger of "rebound" caused by long-term exposure to CNS drugs remains a significant therapeutic concern.

Thus, there remains a need for formulations to achieve optimal plasma level exposure through controlled release formulations that both address the achievement of extended plasma level exposure while also minimizing the potential for increased dependence and withdrawal symptoms of traditional controlled release approaches.

The present invention provides formulations of CNS drugs that achieve a specific therapeutic blood level profile, while avoiding limitations associated with prior formulations. The formulations of the invention may be suitable for use as once-daily chronotherapeutic formulations.

It is to be understood that both the foregoing general description and the following detailed description are exemplary and explanatory only and are not restrictive of the invention, as claimed.

### BRIEF DESCRIPTION OF THE DRAWINGS

Figure 1 is a profile of the mean single dose simulated plasma concentration versus time profile of a delayed onset tramadol formulation with 35% EUDRAGIT® RS-30D.

Figure 2 is a profile of the mean steady state simulated concentration versus time profile of a delayed onset tramadol formulation with 35% EUDRAGIT® RS-30D over 0-120 hr post administration.

Figure 3 is a profile of the mean steady state simulated plasma concentration versus time profile of a delayed onset tramadol formulation with 35% EUDRAGIT® RS-30D over 96-120 hr post administration.

### DESCRIPTION

For drugs of dependence and short half life (*e.g*., less than 8 hours), a deliberate fluctuation in plasma concentrations during the once-daily dosage interval allows for limited constant CNS exposure to the drug, while maintaining a controlled and extended exposure for the major portion of the dosage interval. This profile of extended exposure coupled with a deliberate peak-to-trough fluctuation is desirable to achieve at steady-state, i.e., on continuous repeated dosing for >5 times the half-life.

A simple control of release rate and associated absorption rate of traditional controlled release dosage forms cannot achieve this desired profile as the ability to "control" *in-vivo* input rate may only approximate and trailing input at steady state may blunt the apparent single dose peak-to-trough fluctuation.

The present inventor discovered a delayed onset formulation comprising at least one CNS drug that exhibits an *in vivo* half-life of less than about 8 hours, wherein the formulation exhibits at least one *in vivo* parameter, at steady state following administration to a subject, chosen from: 1) an initial lag in absorption from about 2 to about 6 hours; 2) a peak-to-trough ratio of ≥ about 4:1; 3) a percent fluctuation of ≥ about 100%; and 4) a minimum time cover of ≥ 50% of Cₘₐₓ of at least 8 hours.

As used herein, the term "CNS drug" refers generally to the classes of drugs acting on the central nervous system. Mention may be made, among the CNS drugs, for example, of sedative-hypnotic drugs, anti-seizure drugs, general anesthetics, local anesthetics, skeletal muscle relaxants, antipsychotic agents and lithium, antidepressant agents, antidyskinetics, *e.g*., those CNS drugs associated with Parkinson's and other movement disorders, and opioid analgesics and antagonists.

As used herein, the term "absorption half-life" refers to the time required for 50% of a drug to be absorbed following administration to a subject.

As used herein, the phrase "delayed onset formulation" refers to a pharmaceutical preparation that substantially or completely withholds or impairs delivery of a compound for a specified period of time, *i.e.,* the delay period. Following this delay period, the active ingredient of such formulation begins to be released. Without further impairment, the full amount of the drug may be released rapidly and/or in a controlled release manner. For example, a typical delayed onset release tablet will inhibit release of its active compound until an exterior coating disintegrates or erodes. Once the coating is dissolved, the active compound may be rapidly released into the subject and/or the active compound may be released in a controlled release manner based on the tablet's core formulation, which, like the coating, may result in a "controlled release" depending on the formulation's excipients to further regulate the release of the active compound.

As used herein, the term "elimination half-life" refers to the time required for 50% of a drug to be eliminated following administration to a subject. A "short elimination half-life drug" is one that exhibits an elimination half-life (t_{1/2}) of less than about 8 hours following administration to a subject. Examples of drugs having a short elimination half-life are provided in Table 1. One of skill in the art is familiar with the half-life of any given drug and methods for determining the same.

For example, the elimination half-life of a drug is typically estimated as [In2 / kel], where kel = [(InC1-InC2) / (t2-t1)]. C1 and C2 are concentrations at time t1 and t2, respectively, in the log-linear terminal phase of the plasma concentration versus time curve.

**TABLE 1 - Examples of short elimination half-life drugs.**

| **Drug** | **Half-Life Elimination** **(hours)** |
|---|---|
| tramadol (parent and metabolite) | 5-6 and 7 |
| methylphenidate | 3.5 |
| oxycodone | 0.4 |
| pentazocine | 3.6 |
| metaxolone | 9.2 ± 4.8 (fasting) 2.4 ± 1.2 (after a meal) |
| morphine | 1.5-2.0 |

The term "pharmaceutically acceptable salt" includes salts that are physiologically tolerated by a subject. Such salts are typically prepared from an inorganic and/or organic acid. Examples of suitable inorganic acids include, but are not limited to, hydrochloric, hydrobromic, hydroiodic, nitric, sulfuric, and phosphoric acid. Organic acids may be aliphatic, aromatic, carboxylic, and/or sulfonic acids. Suitable organic acids include, but are not limited to, formic, acetic, propionic, succinic, camphorsulfonic, citric, fumaric, gluconic, lactic, malic, mucic, tartaric, paratoluenesulfonic, glycolic, glucuronic, maleic, furoic, glutamic, benzoic, anthranilic, salicylic, phenylacetic, mandelic, pamoic, methanesulfonic, ethanesulfonic, pantothenic, benzenesulfonic (besylate), stearic, sulfanilic, alginic, galacturonic, and the like.

In some embodiments tramadol is the central acting analgesic used in the present disclosure. A tramadol salt may be selected on the basis of its solubility, as needed to achieve the desired pharmaceutical and/or pharmacokinetic properties in the formulation. Examples of very soluble salts include hydrochloride and sulphate salts. In one embodiment, the analgesic is a hydrochloride salt of tramadol. Solubility considerations may also be used to select particular salts from among the other CNS drugs encompassed by the present invention.

As used herein, the term "pharmaceutically acceptable excipient" includes compounds that are compatible with the other ingredients in a pharmaceutical formulation and not injurious to the subject when administered in acceptable amounts.

As used herein, the phrase "therapeutically effective amount " refers to the amount of a drug compound, or pharmaceutically acceptable salt thereof, that alone and/or in combination with other drugs provides a benefit in preventing, treating, and/or managing at least one condition that may benefit from the properties of that particular drug.

The term "Tₘₐₓ" refers to the time at which the peak level of drug plasma level is attained in a subject following administration of the drug to the subject.

The term "lag-time" refers to the time before the plasma concentration increases about 15% of the difference between the peak and trough concentrations from the trough concentration after administration of the formulation at steady state.

The terms "peak-to-trough fluctuation" or "peak-to-trough ratio" refer to the ratio of the peak plasma concentration to the minimum plasma concentration in a dosing interval at steady-state.

The term "time cover" refers to the duration of time in a dosing interval at steady-state that plasma concentrations are above a minimum concentration defined in this application as 50% of the peak concentration.

The present disclosure is directed to compositions and methods for preventing, treating, and/or managing conditions that are preventable, treatable, and/or manageable with CNS drugs. For example, the present disclosure may be suitable for CNS drugs that exhibit a short elimination half-life following administration to a subject.

In one embodiment, the present invention relates to delayed onset, controlled release formulations comprising at least one short elimination half-life CNS drug, and methods of their use in preventing, treating, and/or managing CNS conditions. In some embodiments, the present disclosure relates to delayed onset, controlled release formulations comprising at least one short elimination half-life CNS drug, and methods of their use, in providing an effective therapy for such conditions while maintaining a controlled and extended exposure of the drug for the dosage interval. In further embodiments, the present invention relates to delayed onset, controlled release formulations comprising at least one short elimination half-life CNS drug, and methods of their use, in providing an effective therapy for such conditions while preventing and/or reducing side-effects, rebound phenomenon, i.e., withdrawal, tolerance and/or dependence.

In some embodiments, the invention relates to delayed onset, controlled release formulations comprising at least one analgesic, and methods of their use in preventing, treating, and/or managing CNS conditions. In some embodiments, the present invention relates to delayed onset, extended release formulations comprising at least one analgesic, and methods of their use, in providing an effective therapy for such conditions while maintaining a controlled and extended exposure of the drug for the dosage interval. In further embodiments, the present disclosure relates to delayed onset, extended release formulations comprising at least one analgesic, and methods of their use, in providing an effective therapy for such conditions while preventing and/or reducing side-effects, rebound phenomenon, tolerance and/or desensitization.

The present formulations overcome at least one deficiency associated with prior art formulations of CNS drugs. For example, the present formulations can avoid or reduce long-term desensitization, rebound phenomena (i.e., withdrawal effects), and/or various undesirable side effects, while maintaining a reliable and reproducible drug plasma profile that is consistent over a course of multiple doses.

The present formulations are suitable for use as chronotherapeutics for once-daily administration. In some embodiments, the chronotherapeutic formulation is a delayed onset formulation comprising at least one CNS drug that exhibits an *in vivo* elimination half-life of less than about 8 hours, wherein the formulation exhibits at least one *in vivo* parameter, at steady state following administration to a subject, chosen from:
1) an initial lag in absorption from about 2 hours to about 6 hours;
2) a peak-to-trough ratio of greater than or equal to about 4:1;
3) a percent fluctuation of greater than or equal to about 100%; and
4) a minimum time cover of ≥ 50% of Cₘₐₓ of at least 8 hours.

The present formulations are designed to satisfy at least one of those parameters such as at least two of those parameters or at least three of those parameters and further for example, all four of those parameters, while taking into account the varying absorption half-life and elimination half-life values of different CNS drugs. For example, the present disclosure may be suitable for using short elimination half-life CNS drugs in chronotherapeutic formulations.

The delay in the release of therapeutic concentrations of the short elimination half-life CNS drug(s) may be from about 2 to about 6 hours, from about 3 to about 6 hours, or from about 3 to about 4 hours, or any hour or fraction of time in between, following administration of the formulation. For example, the present controlled-release formulations may delay release of therapeutic concentrations of the short elimination half-life CNS drug(s) or pharmaceutically acceptable salt thereof for about 2, 3, 4, 5, or 6 hours, or any hour or fraction of time in between, following administration.

Following release of the drug, therapeutic levels of the drug may be maintained for at least 8 hours. Typically, the short elimination half-life CNS drug(s) is maintained at or above the therapeutic level for about 8 to about 20 hours, or any hour or fraction of time in between, measured from the time of administration. Accordingly, the CNS drug(s) is maintained at or above the therapeutic level for about 8, 9, 10, 11, 12, 13, 14, 15, 16, 17, 18, 19 or 20 hours, or any hour or fraction of time in between, measured from the time of administration. In this manner, the present formulations provide therapeutically effective amounts of the drug throughout the day, *i.e.,* a given time period.

The formulations also provide for a "washout phase" by requiring a peak-to-trough ratio of greater than or equal to 4:1 at steady state. As compared to the maximum CNS drug plasma levels attained following release of the drug, the level to which the blood plasma concentration falls during a washout period exhibits a ratio (peak-to-trough) of greater than about 4:1. Thus, the peak-to-trough ratio may be about 4:1, 5:1, 6:1, 7:1, 8:1, 9:1, 10:1, or greater, or any fraction in between.

In so doing, the plasma concentration of the short elimination half-life CNS drug(s) in the blood stream of the subject is allowed to drop below the minimum therapeutic level until the next dose of the drug is administered. In some particular formulations, a washout phase may be provided by the delay phase of a subsequent dosage form. In other words, the plasma levels of short elimination half-life CNS drug(s) in the blood stream of the subject following a first administration are allowed to drop below the minimum therapeutic level and remain there during the delay phase of a subsequently administered dose. A typical washout phase will last from about 1 or less hours to about 8 hours, or any hour or fraction of time in between. Thus, the washout phase may last 0.5, 1, 2, 3, 4, 5, 6, 7, or 8 hours, or any hour or fraction of time in between.

The therapeutically effective level for the short elimination half-life CNS drug(s) may vary depending on the drug being used, the patient, and the condition being treated. In some instances, the therapeutically effective level may be determined empirically by determining a subject's response and titrating a dose as necessary. Such experimentation is routine and within the skill in the art. In one embodiment, where tramadol is provided in the formulation, the daily dose ranges from about 1 mg to about 600 mg, or any number in between, for example, from about 25 mg to about 200 mg such as from about 50 mg to about 100 mg.

By administering the present formulations, a subject receiving treatment can avoid or reduce the effects associated with the withdrawal from the drug (*i.e.,* rebound phenomenon). Likewise, an individual who is already taking a CNS drug formulation may substitute or switch to one of the presently disclosed formulations in order to receive the same benefit. In cases where the subject must intentionally be withdrawn from a CNS drug formulation, but desires to avoid the rebound phenomenon, it is advantageous for the subject to switch to one of the presently disclosed formulations for at least about 7 days before ceasing treatment. This will provide adequate time for the subject to adjust before withdrawal from the drug is permitted.

The methods of the present invention involve administering a pharmaceutically effective amount of at least one short elimination half-life CNS drug, or a pharmaceutically acceptable salt thereof, to a subject in need of such treatment. Mention may be made, of CNS drugs, which may be used in the disclosure, for example, of: sedative-hypnotic drugs such as alprazolam, chlordiazepoxide, clorazepate, clonazepam, diazepam, estazolam, flurazepam, halazepam, lorazepam, midazolam, oxazepam, quazepam, temazepam, triazolam, flumazenil, amobarbital, pentbarbital, phenobarbital and secobarbital; anti-seizure drugs such as carbamazepine, primidone, phenytoin, phenobarbital, ethosuximide and valproate; general anesthetics such as benzodiazepines, opiod analgesics, propofol, etomidate and ketamine; local anesthetics such as cocaine, procaine, tetracaine, benzocaine, lidocaine, mepivacine, bupivacaine, etidocaine, prilocaine and ropivacaine; skeletal muscle relaxants such as atracurium, cisatracurium, doxacurium, metocurine, mivacurium, pancuronium, pipecuronium, rocuronium, succinylcholine, tubocurarine, vecuronium, baclofen, botulium toxin type A, botulium toxin type B, carisoprodol, chlorphenesin, chlorzoxazone, cyclobenzaprine, dantrolene, diazepam, gabapentin, metaxalone, methocarbamol, methylphenidate, orphenadrine, riluzole and tizanidine; antipsychotic agents and lithium such as aliphatic phenothiazines, piperazine phenothiazines, thioxanthene, butylrophenone, dibenzodiazepine, benzisoxazole, thienobenzodiazepine, dibenzothiazepine, dihydroindolone, dihydrocarbostyril and lithium carbonate; antidepressant agents such as amitriptyline, amoxapine, bupropion, citalopram, clomipramine, desipramine, doxepin, fluoxetine, fluvoxamine, imipramine, maprotiline, mirtazapine, nefazodone, nortriptyline, paroxetine, proptriptyline, sertraline, trazodone and venlafaxine; and opioid analgesics and antagonists such as morphine, hydromorphone, oxymorphone, methadone, meperidine, fentanyl, sufentanil, alfentanil, levorphanol, codeine, hydrocodone, oxycodone, propoxyphene, pentazocine, nalbuphine, burenorphine and butorphanol. For example, the at least one CNS drug is chosen from tramadol, oxycodone, metaxolone, methylphenidate, pentazocine, morphine, and combinations thereof.

In some embodiments, the short elimination half-life CNS drug may be chosen from anxiolytics, sedatives, hypnotics, antiepileptics, anesthetics, skeletal muscle relaxants, antipsychotics and lithium, antidepressant agents, antidyskinetics, and opioid analgesics and antagonists. In a particular embodiment, the CNS drug may be tramadol.

The CNS conditions that may be prevented, treated, and/or managed using the inventive compositions and methods include, but are not limited to, anxiety, depression, insomnia, psychosis, mania, pain, attention deficient disorder, phobia, dyskinesia, epilepsy, and combinations thereof.

At least one short elimination half-life CNS drug, or a pharmaceutically acceptable salt thereof, may be provided in a pharmaceutical composition for use according to the present disclosure. Such compositions optionally include at least one pharmaceutically acceptable excipient. Suitable excipients are known to those of skill in the art and are described, for example, in the Handbook of Pharmaceutical Excipients (Kibbe (ed.), 3rd Edition (2000), American Pharmaceutical Association, Washington, D.C.), and Remington's Pharmaceutical Sciences (Gennaro (ed.), 20th edition (2000), Mack Publishing, Inc., Easton, PA), which, for their disclosures relating to excipients and dosage forms, are incorporated herein by reference.

Suitable excipients include, but are not limited to, starches, sugars, microcrystalline cellulose, diluents, granulating agents, lubricants, binders, disintegrating agents, wetting agents, emulsifiers, coloring agents, release agents, coating agents, sweetening agents, flavoring agents, perfuming agents, preservatives, plasticizers, gelling agents, thickeners, hardeners, setting agents, suspending agents, surfactants, humectants, carriers, stabilizers, antioxidants, and combinations thereof.

The pharmaceutical compositions of the disclosure are typically provided in dosage forms that are suitable for administration to a subject by a desired route. A number of suitable dosage forms are described below, but are not meant to include all possible choices. One of skill in the art is familiar with the various dosage forms that are suitable for use in the present disclosure, as described, for example, in *Remington's Pharmaceutical Sciences,* portions of which have been incorporated by reference above. The most suitable route in any given case will depend on the nature and severity of the condition being prevented, treated, and/or managed. The pharmaceutical compositions of the present disclosure may be formulated for administration orally, nasally, rectally, intravaginally, intracisternally, and topically (including buccally and sublingually).

Formulations suitable for oral administration include, but are not limited to, capsules, cachets, pills, tablets, lozenges (which may use a flavored base, usually sucrose and acacia or tragacanth), powders, granules, solutions, suspensions in an aqueous or non-aqueous liquid, oil-in-water or water-in-oil liquid emulsions, elixirs, syrups, pastilles (which may use an inert base, such as gelatin and glycerin, or sucrose and acacia), pastes, and the like.

In solid dosage forms for oral administration (capsules, tablets, pills, powders, granules, and the like), suitable excipients include, but are not limited to, carriers, such as sodium citrate or dicalcium phosphate; fillers or extenders, such as starches, lactose, sucrose, glucose, mannitol, or silicic acid; binders, such as hydroxymethyl-cellulose, alginates, gelatin, polyvinylpyrrolidone, sucrose or acacia; humectants, such as glycerol; disintegrating agents, such as agar, calcium carbonate, potato or tapioca starch, alginic acid, certain silicates, or sodium carbonate; solution retarding agents, such as paraffin; absorption accelerators, such as quaternary ammonium compounds; wetting agents, such as cetyl alcohol or glycerol monostearate; absorbents, such as kaolin and bentonite clay; lubricants, such as talc, calcium stearate, magnesium stearate, solid polyethylene glycols, and sodium lauryl sulfate; coloring agents; buffering agents; dispersing agents; preservatives; and diluents. The aforementioned excipients are given as examples only and are not meant to include all possible choices. Solid compositions may also be employed as fillers in soft and hard-filled gelatin capsules using excipients such as lactose or milk sugars, high molecular weight polyethylene glycols, and the like. Any of these dosage forms may optionally be scored or prepared with coatings and shells, such as enteric coatings and coatings for modifying the rate of release, examples of which are well known in the pharmaceutical-formulating art.

Suitable liquid dosage forms for oral administration include emulsions, microemulsions, suspensions, syrups, and elixirs. These formulations may optionally include diluents commonly used in the art, such as, for example, water or other solvents, solubilizing agents and emulsifiers, including, but not limited to, ethyl alcohol, isopropyl alcohol, ethyl carbonate, ethyl acetate, benzyl alcohol, benzyl benzoate, propylene glycol, 1,3-butylene glycol, oils, glycerol, tetrahydrofurfuryl alcohol, polyethylene glycols, fatty acid esters of sorbitan, and mixtures thereof. In addition, the liquid formulations optionally include adjuvants such as wetting agents, emulsifying and suspending agents, sweetening, flavoring, coloring, perfuming, and preservative agents. Suitable suspension agents include, but are not limited to, ethoxylated isostearyl alcohols, polyoxyethylene sorbitol and sorbitan esters, microcrystalline cellulose, aluminum metahydroxide, bentonite, agar-agar and tragacanth, and mixtures thereof. Liquids may be delivered as-is, or in a carrier, such as a hard or soft capsule or the like.

For rectal or vaginal administration, the composition may be provided as a suppository. Suppositories optionally include one or more non-irritating excipients, for example, polyethylene glycol, a suppository wax, or a salicylate. Such excipients may be selected based on desirable physical properties. For example, a compound that is solid at room temperature but liquid at body temperature will melt in the rectum or vaginal cavity and release the active compound. The formulation may alternatively be provided as an enema for rectal delivery. Formulations suitable for vaginal administration also include pessaries, tampons, creams, gels, pastes, foams, or spray formulations containing such carriers, examples of which are known in the art.

Formulations suitable for topical or transdermal administration include powders, sprays, ointments, pastes, creams, lotions, gels, solutions, patches, and inhalants. Such formulations optionally contain excipients such as animal and vegetable fats, oils, waxes, paraffins, starch, tragacanth, cellulose derivatives, polyethylene glycols, silicones, bentonites, silicic acid, talc, zinc oxide, or mixtures thereof. Powders and sprays may also contain excipients such as lactose, talc, silicic acid, aluminum hydroxide, calcium silicates, and polyamide powder. Additionally, sprays may contain propellants, such as chlorofluoro-hydrocarbons and volatile unsubstituted hydrocarbons, such as butane and propane.

Transdermal patches have the added advantage of providing controlled delivery of the drug into the subject's body. Such dosage forms can be made by dissolving, dispersing, or otherwise incorporating a pharmaceutical composition containing at least one CNS drug or pharmaceutically acceptable salt thereof in a suitable medium, such as an elastomeric matrix material. Absorption enhancers can also be used to increase the flux of the mixture across the skin. The rate of such flux may be controlled by providing a rate-controlling membrane or dispersing the compound in a polymer matrix or gel.

For parenteral administration, such as administration by injection (including, but not limited to, subcutaneous, bolus injection, intramuscular, intraperitoneal, and intravenous), the pharmaceutical compositions may be formulated as isotonic suspensions, solutions, or emulsions, in oily or aqueous vehicles, and may contain formulatory agents such as suspending, stabilizing, or dispersing agents. Alternatively, the compositions may be provided in dry form such as a powder, crystalline, or freeze-dried solid, for reconstitution with sterile pyrogen-ee water or isotonic saline before use. They may be presented, for example, in stele ampoules or vials.

Examples of suitable aqueous and nonaqueous excipients include water, ehanol, polyols (such as glycerol, propylene glycol, polyethylene glycol, and the like), oils, injectable organic esters, and mixtures thereof. Proper fluidity can be maintained, for example, by the use of surFactants.

Those compositions may also contain adjuvants such as preservatives, wetting agents, emulsifying agents, and dispersing agents. Preventing the action of microorganisms may be achieved by including various antibacterial and/or antifungal agents, for example, paraben, chlorobutanol, phenol sorbic acid, and the like. It may also be desirable to include isotonic agents, such as sugars, sodium chloride, and the like in the compositions.

To prolong the therapeutic effect of a drug, it may be desirable to slow the absorption of the drug from a subcutaneous or intramuscular injection. Prolonged absorption of the injectable pharmaceutical form may be brought about by the inclusion of agents that delay absorption, such as aluminum monostearate and/or gelatin. This may also be accomplished by the use of a liquid suspension of crystalline or amorphous material having low solubility. The rate of absorption of the drug then generally depends upon its rate of dissolution, which may depend upon crystal size and crystalline form. Alternatively, delayed absorption of a parenterally-administered form can be accomplished by dissolving or suspending the drug in an oil vehicle.

In addition to the common dosage forms discussed above, the pharmaceutical compositions may also be administered by controlled-release delivery devices, examples of which are well known to those of ordinary skill in the art. Examples of different formulations are provided in U.S. Pat. Nos.: 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566, the disclosures of which, for their discussions of pharmaceutical formulations, are incorporated herein by reference. Advantages of controlled-release formulations may include extended activity of the drug, reduced dosage frequency, decreased side-effects (including rebound phenomena, desensitization, and tolerance), and increased patient compliance. Suitable components (*e.g*., polymers, excipients, etc.) for use in controlled-release formulations, and methods of producing the same, are also described, *e.g*., in U.S. Patent No. 4,863,742, which is incorporated by reference for these purposes.

The release of the active ingredient can be slowed or controlled by using, for example, hydroxypropylmethyl cellulose in varying proportions to provide the desired release profile, other polymer matrices, gels, permeable membranes, osmotic systems, multilayer coatings, microparticles, liposomes, microspheres, or the like, or combinations thereof. Examples of suitable delayed- or controlled-release formulations are known to those of ordinary skill in the art, and may readily be selected for use with the short elimination half-life CNS drug formulations of the present invention. Thus, tablets, capsules, gelcaps, caplets, and the like, that are adapted for controlled-release, may be used in accordance with the presently disclosed methods. The controlled-release of the active ingredient may be triggered or stimulated by various inducers, for example pH, temperature, enzymes, water, or other physiological conditions or compounds.

The controlled-release formulations used in the present methods may include any number of pharmaceutically acceptable excipients. Suitable excipients include, but are not limited to, carriers, such as sodium citrate or dicalcium phosphate; fillers or extenders, such as stearates, silicas, gypsum, starches, lactose, sucrose, glucose, mannitol, talc, or silicic acid; binders, such as hydroxymethyl-cellulose, alginates, gelatin, polyvinyl pyrrolidone, sucrose or acacia; humectants, such as glycerol; disintegrating agents, such as agar, calcium carbonate, potato or tapioca starch, alginic acid, certain silicates, or sodium carbonate; solution retarding agents, such as paraffin; absorption accelerators, such as quaternary ammonium compounds; wetting agents, such as cetyl alcohol or glycerol monostearate; absorbents, such as kaolin and bentonite clay; lubricants, such as talc, calcium stearate, magnesium stearate, solid polyethylene glycols, and sodium lauryl sulfate; stabilizers, such as fumaric acid; coloring agents; buffering agents; dispersing agents; preservatives; organic acids; and organic bases. The aforementioned excipients are given as examples only and are not meant to include all possible choices. Additionally, many excipients may have more than one role, or be classified in more than one group; the classifications are descriptive only, and not intended to limit any use of a particular excipient.

Examples of suitable organic acids include, but are not limited to, adipic acid, ascorbic acid, citric acid, fumaric acid, malic acid, succinic acid, tartaric acid, and mixtures thereof. Suitable organic bases, include, but are not limited to, sodium citrate, sodium succinate, sodium tartrate, potassium citrate, potassium tartrate, potassium succinate, and mix tures thereof. Suitable diluents include, but are not limited to, lactose, talc, microcrystalline cellulose, sorbitol, mannitol, xylitol, fumed silica, stearic acid, magnesium stearate, sodium stearate, and mixtures thereof.

In one embodiment, the controlled-release formulations of the present invention are provided as multiparticulate formulations. At least one short elimination half-life CNS drug or pharmaceutically acceptable salt thereof is typically formed into an active core by applying the compound to a nonpareil seed having an average diameter in the range of about 0.4 to about 1.1 mm or about 0.85 to about 1.00 mm. The drug may be applied with or without additional excipients onto the inert cores, and may be sprayed from solution or suspension using a fluidized bed coater (*e.g.,* Wurster coating) or pan coating system. Alternatively, the drug may be applied as a powder onto the inert cores using a binder to bind it to the cores. Active cores may also be formed by extrusion of the core with suitable plasticizers (described below) and any other processing aids as necessary.

The controlled-release formulations of the present invention comprise at least one polymeric material, which may be water-soluble or water-insoluble. Suitable water-soluble polymers include, but are not limited to, polyvinyl alcohol, polyvinylpyrrolidone, methylcellulose, hydroxypropylcellulose, hydroxypropylmethyl cellulose or polyethylene glycol, and/or mixtures thereof.

Suitable water insoluble polymers include, but are not limited to, ethylcellulose, cellulose acetate cellulose propionate, cellulose acetate propionate, cellulose acetate butyrate, cellulose acetate phthalate, cellulose triacetate, poly (methyl methacrylate), poly (ethyl methacrylate), poly (butyl methacrylate), poly (isobutyl methacrylate), and poly (hexyl methacrylate), poly (isodecyl methacrylate), poly (lauryl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene) high density, poly (ethylene oxide), poly (ethylene terephthalate), poly (vinyl isobutyl ether), poly (vinyl acetate), poly (vinyl chloride), or polyurethane, and/or mixtures thereof.

EUDRAGIT™ polymers (available from Rohm Pharma) are polymeric lacquer substances based on acrylates and/or methacrylates. A suitable polymer that is freely permeable to the active ingredient and water is EUDRAGIT™ RL. A suitable polymer that is slightly permeable to the active ingredient and water is EUDRAGIT™ RS. Other suitable polymers that are slightly permeable to the active ingredient and water, and exhibit a pH-dependent permeability include, but are not limited to, EUDRAGIT™ L, EUDRAGIT™ S, and EUDRAGIT™ E.

EUDRAGIT™ RL and RS are acrylic resins comprising copolymers of acrylic and methacrylic acid esters with a low content of quaternary ammonium groups. The ammonium groups are present as salts and give rise to the permeability of the lacquer films. EUDRAGIT™ RL and RS are freely permeable (RL) and slightly permeable (RS), respectively, independent of pH. The polymers swell in water and digestive juices, in a pH-independent manner. In the swollen state, they are permeable to water and to dissolved active compounds.

EUDRAGIT™ L is an anionic polymer synthesized from methacrylic acid and methacrylic acid methyl ester. It is insoluble in acids and pure water. It becomes soluble in neutral to weakly alkaline conditions. The permeability of EUDRAGIT™ L is pH dependent. Above pH 5.0, the polymer becomes increasingly permeable.

In one embodiment, the polymeric material comprises methacrylic acid co-polymers, ammonio methacrylate co-polymers, or mixtures thereof. Methacrylic acid co-polymers such as EUDRAGIT™ S and EUDRAGIT™ L (Rohm Pharma) are particularly suitable for use in the controlled-release formulations of the present invention. These polymers are gastroresistant and enterosoluble polymers. The polymer films are insoluble in pure water and diluted acids. They dissolve at higher pHs, depending on their content of carboxylic acid. EUDRAGIT™ S and EUDRAGIT™ L can be used as single components in the polymer coating or in combination in any ratio. By using a combination of the polymers, the polymeric material may exhibit a solubility at a pH between the pHs at which EUDRAGIT™ L and EUDRAGIT™ S are separately soluble.

The core may comprise a polymeric material comprising a major proportion (*i.e.,* greater than 50% of the total polymeric content) of at least one pharmaceutically acceptable water-soluble polymers, and optionally a minor proportion (*i.e.,* less than 50% of the total polymeric content) of at least one pharmaceutically acceptable water insoluble polymers.

Alternatively, the core may comprise a polymeric material comprising a major proportion *(i.e.,* greater than 50% of the total polymeric content) of at least one pharmaceutically acceptable water insoluble polymers, and optionally a minor proportion (*i.e*., less than 50% of the total polymeric content) of at least one pharmaceutically acceptable water-soluble polymers. The formulations may optionally contain a coating membrane partially or completely surrounding the core, comprising a major proportion of at least one pharmaceutically acceptable film-forming, water-insoluble polymers, and optionally a minor proportion of one or more pharmaceutically acceptable film-forming, water-soluble polymers. The water insoluble polymer may form an insoluble matrix having a high or low permeability to the CNS drug(s).

In one embodiment, the polymeric material comprises methacrylic acid co-polymers, ammonio methacrylate co-polymers, or mixtures thereof. Methacrylic acid co-polymers such as EUDRAGIT™ S and EUDRAGIT™ L are particularly suitable for use in the controlled-release formulations of the present invention. These polymers are gastroresistant and enterosoluble polymers. The polymer films are insoluble in pure water and diluted acids. They dissolve at higher pHs, depending on their content of carboxylic acid. EUDRAGIT™ S and EUDRAGIT™ L can be used as single components in the polymer coating or in combination in any ratio. By using a combination of the polymers, the polymeric material may exhibit a solubility at a pH between the pHs at which EUDRAGIT™ L and EUDRAGIT™ S are separately soluble.

Ammonio methacrylate co-polymers such as EUDRAGIT™ RS and EUDRAGIT™ RL are also suitable for use in the controlled-release formulations of the present disclosure. Those polymers are insoluble in pure water, dilute acids, buffer solutions, or digestive fluids over the entire physiological pH range. The polymers swell in water (and digestive fluids independently of pH). In the swollen state they are permeable to water and dissolved actives. The permeability of the polymers depends on the ratio of ethylacrylate (EA), methyl methacrylate (MMA), and trimethylammonioethyl methacrylate chloride (TAMCI) groups in the polymer. Those polymers having EA:MMA:TAMCI ratios of 1:2:0.2 (EUDRAGIT™ RL) are more permeable than those with ratios of 1:2:0.1 (EUDRAGIT™ RS). Polymers of EUDRAGIT™ RL are insoluble polymers of high permeability. Polymers of EUDRAGIT™ RS are insoluble films of low permeability.

The ammonio methacrylate co-polymers may be combined in any desired ratio. For example, the ratio of EUDRAGIT™ RS: EUDRAGIT™ RL (90:10) may be used. The ratios may be adjusted to provide a delay in release of the drug. For example, the ratio of EUDRAGIT™ RS: EUDRAGIT ™ RL may be about 100:0 to about 80:20, about 100:0 to about 90:10, or any ratio in between. In such formulations, the less permeable polymer EUDRAGIT™ RS would generally comprise the majority of the polymeric material.

The ammonio methacrylate co-polymers may be combined with the methacrylic acid co-polymers within the polymeric material in order to achieve the desired delay in release of the drug. Ratios of ammonio methacrylate co-polymer (*e.g*., EUDRAGIT™ RS) to methacrylic acid co-polymer in the range of about 99:1 to about 20:80 may be used. The two types of polymers may also be combined into the same polymeric material, or provided as separate coats that are applied to the core.

In addition to the EUDRAGIT™ polymers described above, a number of other copolymers may be used to create a delay in drug release. These include methacrylate ester co-polymers (*e.g*., EUDRAGIT™ NE™ 30D). Further information on the EUDRAGIT™ polymers is to be found in "Chemistry and Application Properties of Polymethacrylate Coating Systems," in Aqueous Polymeric Coatings for Pharmaceutical Dosage Forms, ed. James McGinity, Marcel Dekker Inc., New York, pg 109-114).

The polymeric material typically comprises at least one soluble excipient so as to increase the permeability of the polymeric material. Suitably, the soluble excipient may be chosen from among a soluble polymer, a surfactant, an alkali metal salt, an organic acid, a sugar, and a sugar alcohol. Such soluble excipients include, for example, polyvinyl pyrrolidone, polyethylene glycol, sodium chloride, surfac tants such as sodium lauryl sulfate and polysorbates, organic acids such as acetic acid, adipic acid, citric acid, fumaric acid, glutaric acid, malic acid, succinic acid, and tartaric acid and sugars such as dextrose, fructose, glucose, lactose and sucrose, and sugar alcohols such as lactitol, maltitol, mannitol, sorbitol and xylitol, xanthan gum, dextrins, and maltodextrins. In some particular embodiments, polyvinyl pyrrolidone, mannitol, and/or polyethylene glycol are the soluble excipients. The soluble excipient is typically used-in an amount of from about 1 % to about 10% by weight, relative to the total dry weight of the polymer.

The polymeric material can also include at lest one auxiliary agent such as a filler, a plasticizer, and/or an anti-foaming agent. Representative fillers include talc, fumed silica, glyceryl monostearate, magnesium stearate, calcium stearate, kaolin, colloidal silica, gypsum, micronized silica, and magnesium trisilicate. The quantity of filler used typically ranges from about 2% to about 300% by weight, and may range from about 20 to about 100%, based on the total dry weight of the polymer. In one embodiment, talc is the filler.

The coatings can also include a material that improves the processing of the polymers. Such materials are generally referred to as plasticizers and include, for example, adipates, azelates, benzoates, citrates, isoebucates, phthalates, sebacates, stearates, and glycols. Representative plasticizers include acetylated monoglycerides, butyl phthalyl butyl glycolate, dibutyl tartrate, diethyl phthalate, dimethyl phthalate, ethyl phthalyl ethyl glycolate, glycerin, ethylene glycol, propylene glycol, triacetin citrate, triacetin, tripropinoin, diacetin, dibutyl phthalate, acetyl monoglyceride, polyethylene glycols, castor oil, triethyl citrate, polyhydric alcohols, acetate esters, gylcerol triacetate, acetyl triethyl citrate, dibenzyl phthalate, dihexyl phthalate, butyl octyl phthalate, diisononyl phthalate, butyl octyl phthalate, dioctyl azelate, epoxidised tallate, triisoctyl trimellitate, diethylhexyl phthalate, di-n-octyl phthalate, di-i-octyl phthalate, di-i-decyl phthalate, di-n-undecyl phthalate, di-n-tridecyl phthalate, tri-2-ethylhexyl trimellitate, di-2-ethylhexyl adipate, di-2-ethylhexyl sebacate, di-2-ethylhexyl azelate, dibutyl sebacate, glyceryl monocaprylate, and glyceryl monocaprate. In one embodiment, the plasticizer is dibutyl sebacate. The amount of plasticizer used in the polymeric material typically ranges from about 10% to about 50%, for example, about 10, 20, 30, 40, or 50%, relative to the weight of the dry polymer.

In one embodiment, the anti-foaming agent is simethicone. The amount of anti-foaming agent used typically comprises from about 0% to about 0.5% of the final formulation.

The amount of polymer to be used in controlled-release formulations is typically adjusted to achieve the desired drug delivery properties, including the amount of drug to be delivered, that rate, timing, and location of drug delivery, the time delay of drug release, and the size of the multiparticulates in the formulation. The amount of polymer applied typically provides about a 10% to about 100% weight gain to the cores. In one embodiment, the weight gain from the polymeric material is about 25% to about 70%.

The combination of all solid components of the polymeric material, including co-polymers, fillers, plasticizers, and optional excipients and processing aids, typically provides about a 10 to about 450% weight gain on the cores. In one embodiment, the weight gain is about 30 to about 160%.

The polymeric material may be applied by any known method, for example, by spraying using a fluidized bed coater (*e.g*., Wurster coating) or pan coating system.

The coated cores are typically dried or cured after application of the polymeric material. Curing means that the multiparticulates are held at a controlled temperature for a time sufficient to provide stable release rates. Curing may be performed, for example, in an oven or in a fluid bed drier. Curing may be carried out at any temperature above room temperature.

A sealant or barrier may be applied to the polymeric coating. A sealant or barrier layer may also be applied to the core prior to applying the polymeric material. The sealant or barrier layer does not modify the release of short elimination half-life CNS drug(s) significantly. Suitable sealants or barriers are permeable or soluble agents such as hydroxypropyl methylcellulose, hydroxypropyl cellulose, hydroxypropyl ethylcellulose, and xanthan gum. Hydroxypropyl methylcellulose is particularly useful in this regard.

Other agents may be added to improve the processability of the sealant or barrier layer. Such agents include talc, colloidal silica, polyvinyl alcohol, titanium dioxide, micronized silica, fumed silica, glycerol monostearate, magnesium trisilicate, magnesium stearate, or a mixture thereof. The sealant or barrier layer may be applied from solution (*e.g*., aqueous) or suspension using any known means, such as a fluidized bed coater (*e.g*., Wurster coating) or pan coating system. Suitable sealants or barriers include, for example, OPADRY WHITE Y-1-7000 and OPADRY OY/B/28920 WHITE, both of which are available from Colorcon Limited, England.

The present disclosure also provides an oral dosage form comprising a multiparticulate CNS drug formulations-as hereinabove defined, in the form of caplets, capsules, particles for suspension prior to dosing, sachets, or tablets. When the dosage form is in the form of tablets, the tablets may be disintegrating tablets, fast dissolving tablets, effervescent tablets, fast melt tablets, and/or mini-tablets. The dosage form can be of any shape suitable for oral administration of a drug, such as spheroidal, cube-shaped oval, or ellipsoidal. The dosage forms may be prepared from the multiparticulates in a manner known in the art and may include additional pharmaceutically acceptable excipients, as desired.

The thickness of the polymer in the formulations, the amounts and types of polymers, and the ratio of water-saluble polymers to water-insoluble polymers in the controlled-release formulations are generally selected to achieve a desired release profile of the CNS drug(s). For example, by increasing the amount of water insoluble-polymer relative to the water soluble-polymer, the release of the drug may be delayed or slowed.

The amount of the drug administered, as well as the dose frequency, will vary depending on the particular dosage form used and the route of administration. The amount and frequency of administration will also vary according to the age, body weight, and response of the individual subject. A competent physician can readily determine typical dosing regimens without undue experimentation. It is also noted that the clinician or treating physician will know how and when to interrupt, adjust, or terminate therapy in conjunction with individual subject response.

In general, the total daily dosage for treating, preventing, and/or managing the CNS conditions described herein is from about 0.1 mg to about 10,000 mg of at least one CNS drug or pharmaceutically acceptable salt thereof. One of skill in the art is familiar with the recommended starting dosage amounts for any particular drug. In some embodiments, the CNS drug or pharmaceutically acceptable salt thereof is the analgesic tramadol, which may be provided in an amount from about 25 mg to about 200 mg, or from about 30 mg to about 150 mg, or from about 35 mg to about 125 mg, or from about 40 mg to about 100 mg, or from about 45 mg to about 75 mg, or any fraction in between. A single dose may be formulated to contain about 5, 10, 12.5, 25, 50, 100, 200, or 400 mg of tramadol, or any amount in between. In one embodiment, the analgesic(s), or pharmaceutically acceptable salt(s) thereof, comprise about 0.5 to about 20%, about 0.5 to about 8%, or about 0.5 to about 4% of the total weight of the formulation.

Any of the pharmaceutical compositions and dosage forms described herein may further comprise at least one additional pharmaceutically active compound or pharmaceutically acceptable salt thereof. Such compounds may be included to treat, prevent, and/or manage the same condition being treated, prevented, and/or managed with the drug that is already present, or a different condition altogether. Those of skill in the art are familiar with examples of the techniques for incorporating additional active ingredients into compositions comprising CNS drugs. Alternatively, such additional pharmaceutical compounds may be provided in a separate formulation and co-administered to a subject with a CNS drug formulation according to the present invention. Such separate formulations may be administered before, after, or simultaneously with the administration of the CNS drug formulations of the present disclosure. In one embodiment, the CNS formulation is co-administered with at least one other compound including, but not limited to: acetaminophen, ibuprofen, naproxen, rofecoxib, celecoxib and diclofenac.

Other than in the examples, or where otherwise indicated, all numbers expressing quantities of ingredients, reaction conditions, and so forth used in the specification and claims are to be understood as being modified in all instance by the term "about." Accordingly, unless indicated to the contrary, the numerical parameters set forth in the following specification and attached claims are approximations that may vary depending upon desired properties sought to be obtained herein. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope are approximations, the numerical values set forth in the specific examples are reported as precisely as possible. Any numerical value, however, inherently contain certain errors necessarily resulting from the standard deviation found in their respective testing measurements.

The present disclosure is further illustrated by reference to the following example. It will be apparent to those skilled in the art that many modifications, both to the materials and methods, may be practiced without departing from the purpose and scope of the present disclosure.

### EXAMPLES

### EXAMPLE 1 - DELAYED ONSET TRAMADOL FORMULATIONS

Tramadol pellets via a powder layering technique using Glatt GPCG 1.1 rotor system were prepared according to the following description. Each of the below listed ingredients was weighed.

| **Ingredient** | **Quantity (g)** |
|---|---|
| Tramadol HCl | 1000.0 |
| Talc | 5.3 |
| Aerosil 200 | 15.9 |
| Kollidon | 48.7 |

Tramadol HCl and the listed excipients were mixed by hand in a plastic bag for approximately 5 minutes. Then, the mixture was passed through a 0.4 mm sieve and mixed again in the plastic bag.

500 g of cellulose starter pellets of 700-1000 µm were rotated in a rotor container. The powder mixture of tramadol HCl was transferred in small portions from a powder feeder into the rotor container. By controlled spraying of demineralized water, the powder mixture was bound onto the starter pellets.

To obtain a sustained release coating of the tramadol pellets, each of the below listed ingredients was prepared:

| **Ingredient** | **Quantity (%)** |
|---|---|
| EUDRAGIT® RS 30 D | 35%, 40%, and 50% |
| Talc | 50% |
| Triethyl Citrate (TEC) | 20% |
| Water | (acc. to 25% solid content) |

The talc and the TEC were homogenized for approximately 10 minutes by means of an Ultra Turrax. Afterwards, the suspension was poured into the EUDRAGIT® RS 30D while stirring with a magnetic stirrer. Once a polymer solution was obtained, it was sprayed onto the tramadol pellets. After the required amount of polymer solution was applied, the polymer-coated tramadol pellets were dried in a fluid coating machine.

The tramadol pellets were coated with varying levels of the polymer solution. For example, the coating was applied to the Tramadol pellets at 35%, 40% and 50% of coating polymer thickness (i.e., percentage weight gain on the tablet coat). The coating was applied onto the tramadol pellets using suitable coating equipment. The theoretical content of the tramadol pellets were calculated as follows:

| **Description** | **Amt. of core material (g)** | **Amt. of Solids Applied (g)** | **Amt. of Tramadol/Polymer Applied (g)** | **Amt. of Solids Applied (%)** | **Amt. of Tramadol/Polymer applied (%)** | **Theoretical Tramadol content** |
|---|---|---|---|---|---|---|
| Uncoated Tramadol | 500.0 | 1069.8 | 1000.0 | 215.0 | 200.0 | 63.7 |
| Tramadol with 35% RS 30D | 980.0 | 583.1 | 343.0 | 59.5 | 35.0 | 39.9 |
| Tramadol with 40% RS 30D | 980.0 | 666.4 | 392.0 | 68.0 | 40.0 | 37.9 |
| Tramadol with 50% RS 30D | 500.0 | 425.0 | 250.0 | 85.0 | 50.0 | 34.4 |

### EXAMPLE 2 - DISSOLUTION ANALYSIS OF DELAYED ONSET TRAMADOL

### FORMULATIONS

*In vitro* dissolution tests were performed on the delayed onset tramadol formulations prepared in Example 1 using the following parameters: USP (11); paddle @ 75 RPM; media: phosphate buffer at pH 6.8 or higher at 37°C; UV absorbance at 270 nm.

Samples were collected at the following time points (hr): 1, 2, 4, 6, 9, 12, 18, and 24, while being subjected to dissolution testing. The *in vitro* dissolution profile for the delayed onset Tramadol formulations are shown below:

Tramadol with 35% EUDRAGIT® RS 30D

| **Time (hr)** | **Average (%)** |
|---|---|
| 0 | 0.00 |
| 1 | 2.02 |
| 2 | 2.41 |
| 4 | 17.07 |
| 6 | 67.44 |
| 9 | 88.25 |
| 12 | 93.42 |
| 18 | 97.50 |
| 24 | 98.05 |

Tramadol with 40% EUDRAGIT® RS 30D

| **Time (hr)** | **Average (%)** |
|---|---|
| 0 | 0.00 |
| 1 | 2.36 |
| 2 | 2.39 |
| 4 | 7.42 |
| 6 | 39.29 |
| 9 | 80.79 |
| 12 | 90.52 |
| 18 | 95.80 |
| 24 | 97.80 |

Tramadol with 50% EUDRAGIT® RS 30D

| **Time (hr)** | **Average (%)** |
|---|---|
| 0 | 0.00 |
| 1 | 0.57 |
| 2 | 0.81 |
| 4 | 0.79 |
| 6 | 1.91 |
| 9 | 78.66 |
| 12 | 55.35 |
| 18 | 83.49 |
| 24 | 89.10 |

Based on the results detailed above, it was determined that the batch of tramadol pellets coated with 35% EUDRAGIT® RS 30D was the batch that provided the closest likeness to the dissolution profile sought to be achieved. As such, a further batch was prepared and additional time points taken to capture the lag time found in the dissolution profile. The following *in vitro* dissolution results were obtained from the additional batch of tramadol pellets with 35% EUDRAGIT® RS 30D:

| **Time (hr)** | **Average (%)** |
|---|---|
| 0 | 0.00 |
| 0.5 | 0.00 |
| 1 | 0.00 |
| 2 | 0.25 |
| 3 | 6.88 |
| 4 | 34.21 |
| 6 | 77.13 |
| 9 | 89.91 |
| 12 | 92.20 |
| 18 | 94.43 |
| 24 | 95.93 |

The data provided for the Tramadol with 35% EUDRAGIT® RS 30D formulations confirms that the desired sustained release formulation was achieved.

### EXAMPLE 3 - PHARMACOKINETIC SIMULATIONS OF DELAYED ONSET

### TRAMADOL FORMULATION WITH 35% EUDRAGIT® RS 30D

The aim of the pharmacokinetic simulations was to predict the *in vivo* Tramadol plasma concentrations over a chosen time course at single dose and at steady state based on the *in vitro* generated dissolution profiles from the delayed onset Tramadol formulation with 35% EUDRAGIT® RS 30D.

The mean *in vitro* release profile for the two batches of the delayed onset Tramadol formulation with 35% EUDRAGIT® RS 30D provided in Example 2 was used. The mean values were as follows:

| **Time (hr)** | **Average (%)** |
|---|---|
| 0.5 | 1.16 |
| 1 | 1.05 |
| 2 | 1.33 |
| 3 | 7.14 |
| 4 | 33.96 |
| 6 | 77.59 |
| 9 | 90.92 |
| 12 | 93.84 |
| 18 | 95.86 |
| 24 | 96.96 |

Pharmacokinetic simulations were undertaken using specialized pharmacokinetic software, PDₓ₋IVIVC® Version 1.0 (Globomax Service Group, Maryland, USA). The simulations were based on the following assumptions: (1) UIR (unit impulse response) based on a 1 compartmental pharmacokinetic model; (2) volume of distribution of 252L (3.6 L per kg based on a 70 kg individual); (3) terminal elimination rate constant of 0.1100h⁻¹ (terminal half life of 6.3 hours); (4) bioavailability of controlled release formulation was assumed equal to that of an immediate release formulation; (5) bioavailability following oral administration was assumed as 75%; and (6) complete absorption was assumed throughout the GI tract.

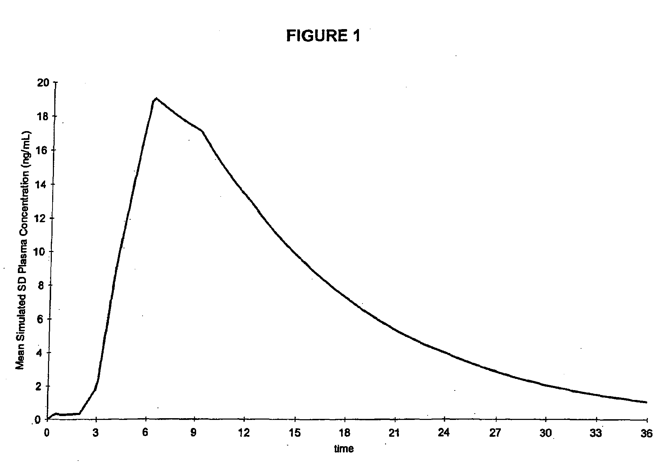

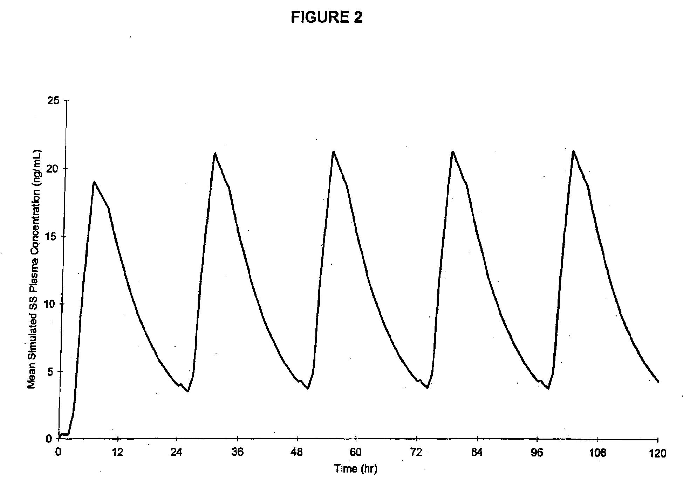

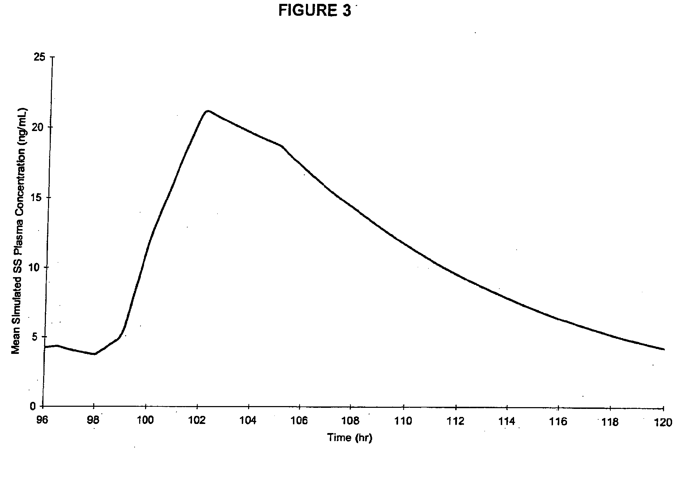

The simulated single dose plasma concentration versus time data profile is presented in Figure 1. The steady state pharmacokinetic simulated plasma concentration versus time profile was simulated by noncompartmental superposition, as illustrated in Figure 2. The mean steady state simulated plasma concentration versus time profile for 96-120 hr post-administration is provided in Figure 3.

Based on the noncompartmental analysis, the following steady state pharmacokinetic parameters were determined:
AUC_{(0-inf)} (ng/mL.hr) = 305.17
AUC₍₀₋ₜₐᵤ₎ (ng/mL.hr) = 262.39
Cₘₐₓ (ng/mL) = 21.17
C_{avg} (ng/mL) = 10.93
Cₘᵢₙ (ng/mL) = 3.76
Tmax (hr) = 102.09
Tₘᵢₙ (hr) = 97.98
Peak to trough ratio = 5.63
Fluctuation % = 159.23
Time Cover (hr)* = 11.10
* Time duration that plasma concentrations are in excess if 50% C_{avg} concentration.

The pharmacokinetic simulations analysis suggests that the predicted *in vivo* profile would have: (1) an initial lag in absorption of approximately 3 hours; (2) a peak-to-trough ratio of 5.6; a percent fluctuation of 159%; and a minimum time cover of greater than or equal to 50% of C_{avg} of at least 11 hours. From the simulated pharmacokinetic parameters, the tramadol delayed onset formulation with 35% EUDRAGIT® RS-30D meets the peak-to-trough fluctuation, while achieving the minimum duration of therapeutic time cover.

## Claims

1. An oral delayed onset formulation comprising a CNS drug or the pharmaceutically acceptable salt thereof that exhibits an *in vivo* elimination half-life of less than about 8 hours, wherein the delayed onset formulation exhibits at least three of the *in vivo* parameters, at steady state following administration to a subject, selected from:
(a) an initial lag in absorption from 2 hours to 6 hours;
(b) a peak-to-trough ratio greater than or equal to 4 : 1;
(c) a percent fluctuation of greater than or equal to 100%; and
(d) a minimum time cover of greater than or equal to 50% of Cₘₐₓ of at least 8
hours;
wherein the CNS drug the pharmaceutically acceptable salt thereof is selected from the group consisting of tramadol, methylphenidate. oxycodone, pentazocine, metaxolone, morphine, and combinations thereof, the formulation being a multiparticulate formulation comprising active cores obtained by applying the CNS drug onto inert cores or by extruding cores, and the cores being coated with a polymeric material.

2. The formulation according to claim 1, wherein the inert core is a non-pareil seed having an average diameter in the range of about 0.4 to about 1.1 mm.

3. The formulation according to claim 2, wherein the average diameter is about 0.85 to about 1.00 mm.

4. The formulation according to any one of the preceding claims, wherein the polymeric material is selected from water-soluble polymers, water-insoluble polymers and combinations thereof.

5. The formulation according to claim 4, wherein the water-soluble polymers are selected from the group consisting of polyvinyl alcohol, polyvinylpyrrolidone, methyl cellulose, hydroxypropyl cellulose, hydroxypropyl methyl cellulose and polyethylene glycol.

6. The formulation according to claim 4, wherein the water-insoluble polymers are selected from the group consisting of ethylcellulose, cellulose acetate, cellulose propionate, cellulose acetate propionate, cellulose acetate butyrate, cellulose acetate phthalate, cellulose triacetate, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), poly(ethylene), poly(ethylene), poly(propylene), poly(ethylene oxide), poly(ethylene terephthalate), polyvinyl isobutyl ether), polyvinyl acetate), polyvinyl chloride), polyurethane, and mixtures thereof.

7. The formulation according to any one of claims 1 - 4, wherein the polymeric material comprises methacrylic acid co-polymers, ammonio methacrylate co-polymers, or mixtures thereof.

8. The formulation according to claim 7, wherein the polymeric material is selected from Eudragit S, Eudragit L, Eudragit E, Eudragit RS and Eudragit RL.

9. The formulation according to any one of the preceding claims, wherein the polymeric material comprises a soluble excipient in an amount of 1% to about 10 % by weight, based on the total dry weight of the polymer material.

10. The formulation according to any one of the preceding claims, wherein the polymeric material comprises a filler in an amount of 2% to 300% by weight, based on the total dry weight of the polymer material.

11. The formulation according to any one of the preceding claims, wherein the polymeric material comprises a plasticizer in an amount of 10% to 50% by weight, based on the total dry weight of the polymer material.

12. The formulation according to any one of the preceding claims, wherein the formulation comprises a sealant or barrier layer.

13. The formulation according to any one of the preceding claims, wherein the CNS drug or the pharmaceutically acceptable salt thereof is tramadol or the hydrochloride salt of tramadol.

14. A delayed onset formulation according to any one of Claims 1 - 13 for use in treating at least one CNS condition.

15. The delayed onset formulation according to Claim 14, wherein the formulation is administered one time per day.

16. The delayed onset formulation according to Claim 14 or Claim 15, wherein the at least one CNS condition is chosen from anxiety, depression, insomnia, psychosis, mania, pain, attention deficient disorders, phobias, epilepsy, and combinations thereof.

17. The delayed onset formulation according to any one of Claims 14 - 16, wherein the amount of tramadol administered is from about 25 mg to about 200 mg per day, preferably from about 50 mg to about 100 mg per day.

18. The delayed onset formulation according to any one of Claims 1 - 13 for reducing the effects of the rebond phenomena in a subject that is to be withdrawn from at least one CNS drug.

19. The delayed onset formulation according to any one of Claims 1 - 13 for decreasing long-term desensitization to a CNS drug therapy.
